Europäisches Patentamt

European Patent Office

Office européen des brevets

(11) Publication number: **0 563 383 A1**

## EUROPEAN PATENT APPLICATION
### published in accordance with Art. 158(3) EPC

(21) Application number: 91920729.0

(22) Date of filing: 04.12.91

(86) International application number:
**PCT/KR91/00032**

(87) International publication number:
**WO 92/10562 (25.06.92 92/14)**

(51) Int. Cl.5: **C12N 1/14**, C12P 21/00,
//(C12N1/14,C12R1:645),
(C12P21/00,C12R1:645)

(30) Priority: **04.12.90 KR 1987790**

(43) Date of publication of application:
**06.10.93 Bulletin 93/40**

(84) Designated Contracting States:
**AT BE CH DE DK ES FR GB GR IT LI LU MC NL SE**

(71) Applicant: **IL YANG PHARM. IND. CO., LTD.**
**24-5 Hawolgogdong,**
**Sungbug-gu**
**Seoul 136-132(KR)**

(72) Inventor: **LEE, Kwon, Haeng**
**699-10,Keumjung-dong**
**Gunpo-shi**
**Kyoungki-do(KR)**
Inventor: **CHUNG, Hoon 165, Okuem-dong**

**Songpa-gu**
**Seoul(KR)**
Inventor: **LEE, Choon, Woo 84-6, Machun**
**2-dong**
**Songpa-gu**
**Seoul 138-122(KR)**
Inventor: **CHUNG, Chun, Hee 249-6,**
**Gaebong-dong**
**Guro-gu**
**Seoul 152-090(KR)**

(74) Representative: **Weisert, Annekäte, Dipl.-Ing.**
**Dr.-Ing. et al**
**Patentanwälte**
**Kraus Weisert & Partner**
**Thomas-Wimmer-Ring 15**
**D-80539 München (DE)**

(54) **(GANODERMA LUCIDIUM) IY009 WHICH PRODUCES PROTEOGLYCAN (G009) HAVING EFFECT OF ENHANCING ANTITUMOR IMMUNITY.**

(57) A strain, *Ganoderma lucidium*, which produces a proteoglycan (G009) which has an effect of enhancing antitumor immunity and contains $\beta$-glucose, $\alpha$-glucose, galactose, $\alpha$-mannose and fructose as the sugar components and glycine, leucine, alanine, histidine, arginine, valine, aspartic acid, threonine, isoleucine, seryl-leucine, glutamic acid, tyrosine, proline, phenylalanine and methionine as the protein components, said proteoglycan being separated from the culture medium of *Genoderma lucidium*.

EP 0 563 383 A1

Brief description of the drawings

Figure 1 is a saccharide analysis chromatogram of proteoglycan G009 according to the present invention.

Figure 2 is an amino acid chromatogram of proteoglycan G009 according to the present invention.

Figure 3 is an I.R. analysis diagram of proteoglycan G009 according to the present invention.

Figure 4 is a microphotograph showing the mycelia of a strain IY 009 according to the present invention.

Figure 5 is a diagram drawn to compare the specificity of a strain IY 009 according to the present invention.

Detailed description of the invention

The present invention relates to a strain which produces a proteoglycan effective in enhancing antitumor immunity.

In order to search for a new proteoglycan which has the effect of promotion of antitumor immunity, the present inventors collected a large number of basidiomycetes and attempted to separate a proteoglycan produced by those basidiomycetes. In sequel thereto, it was found that strains of the genus of Ganoderma which belongs to basidiomycetes, picked up in the area of the Dooryoon Mountains located in the South Cholla Province, produces a proteoglycan which has the effect of promoting antitumor immunity when cultured in a proper culture medium. After the produced proteoglycan was separated and its physicochemical and biophysical properties was examined, it was designated as proteoglycan G009 which has the effect of promoting antitumor immunity and the strain which produces it was designated as Ganoderma lucidum IY 009 ( a number of deposition to the Korean Species Association is KFCC-107090 dated Oct. 5, 1990).

The present invention relates to the strain IY 009 which produces G009. Although researches have been conducted in such medicinal components as antibacterial, hallucinative, toxicant and cholesterol-lowering components from the basidiomycetes belonging to a higher fungus, earnest study is now given to an antitumor component and an immunity improvement action therefrom, and so basidiomycetes are rising in importance. Thus, those strains which secrete such matter as contains an antitumor component and improves an immunity function was separated and cultured in liquid to make a study of its pharmacological effect.

During the study, a mutant strain which has an antitumor effect and an immunity improvement function that are wore excellent than the species belonging to the genus of Ganoderma which has been identified until now was separated and an in-liquid mycelia culture method was established and then the antitumor effect and immunity improvement function of those components extracted from the cultured mycelia were confirmed.

The present invention will now be described in detail according to the following example and experiments.

Example

Separation of proteoglycans

1) Strain :

The ganoderma lucidum starins were collected in the area of the Dooryoon Mountains located in the South Cholla Province and separated with identification.

2) Preserved culture medium :

An inclined culture medium for potato dextrose agar(PDA) ; 39g of potato dextrose culture medium-(Difico, USA) was made into 1 liter by dissolving it in distilled water and it was made into an inclined culture medium after high-pressure sterilization for 20 minutes at 121°C.

A culture medium for in-liquid culture :

50g of glucose, 20g of peptone, 0.87g of $KH_2PO_4$, 0.5g of $MgSO_4 \cdot 7H_2O$, 10mg of $FeCl_2 \cdot 6H_2O$, 7mg of $MnCl_2 \cdot 4H_2O$, 10mg of $ZnSO_2 \cdot 5H_2O$ and 4mg of $ZnCl$ were made into 1 liter by adding distilled water thereto. It was sterilized for 20 minutes at 121°C by adjusting the pH value to 5.5.

2

3) Culture :

The strains kept in custody were transplanted into the inclined culture media for PDA and grown for 7 days at 25±1°C. Then, the grown myclia were separated in an aseptic way and put into 100ml of culture medium for in-liquid culture and triturated with a microblender for 15 seconds. It was moved into a 500ml-triangular flask and shake-cultured for 10 days until the mucelium formed a mature mycetome with a diameter of about 5mm by 180rpm at 25±1°C.

After the mycetome was triturated with a microblender for 10 seconds, it was inoculated 5%(V/V) by 5% into a 500ml-triangular flask containing a 100ml-culture medium for in-liquid culture and shake-cultured for 10 days under the same conditions as stated above.

The cultured mycelia were made into species by triturating them again for 10 seconds and the 100ml-culture medium for in-liquid culture was put into the 500ml-triangular flask and the species was inoculated thereinto 5% by 5%. Then, it was shake-cultured by 170rpm for 7 days in an orbital shaker(the radius of revolution: 1 inch, Vision Science Co.) at 25±1°C.

4) Extraction and separation of proteoglycans

After the whole of culture-fininshed solution was centrifugally separated for 15 minutes by 6000rpm, only mycelia were taken and digested into a twofold 2.5N NaOH solution. It was left as it was for 24 hours at room temperature and centrifugally separated for 15 minutes by 6000rpm. After a supernatant solution was neutralized to pH 7.4 with glacial acetic acid, it was dialyzed for 3 days with a visking tube(Sigma, USA). After dialysis, it was concentrated and twofold ethanol was added thereto. After it was left as it was for 24 hours at 4°C, it was centrifugally separated by 6000rpm for 15 minutes and a precipitate was obtained therefrom. The precipitate was dissolved in deionized water and its supernatant solution was concentrated through centrifugal separation for 1 minutes by 6000rpm and a proteoglycan was separated therefrom by lyophilizing the concentrated solution.

5) Purification of proteglycan

12.5g of precipitate taken after the above sample was dissolved in water and excessive ethanol was added thereto was dissolved in water again. After this sample solution was applied to DEAE-cellulose(Cl-form)column(3cm x 60cm), it was centrifugally separated by adding the same amount of methanol to 200ml of elution fraction obtained by elution with water and G1(2.1g) was obtained by washing a precitate in ethanol and dessicating it under reduced pressure. G2(0.42g) was obtained by the decompressed dessication of a precipitate obtained after a supernatant solution(400ml) was centrifugally separated by adding 400ml of ethanol thereto. G3(1.1g) was obtained by the decompressed essication of a precipitate obtained after a supernatant solution(400ml) was centrifugally separated by adding ethanol (800ml) again to the supernatant solution(800ml) remaining after G2 was removed.

G1 was eluted with by dissolving it in water and applying it to Sephatex G-100. G4(1.5g) was obtained by liophilizing the fraction of a tube No.25-33 and separated by the decompressed dessication of G5(0.3g) including the fraction of a tube No.34-44. After G4 was dissolved in water and the same amount of a solution mixed in the same amount was added to 0.15M cetavelon(cethytrimethyl ammonium bromide) and 0.1M borate buffer(pH 8.0), a precipitate(G7) and a supernatant solution(G6) were taken by adjusting pH to 9.0 with 0.5M NaOH. After G7 was dissolved in water by adding 2M glacial acetic acid, a precipitate was obtained by adding twofold methanol. After the precipitate was washed in methanol and aceton, it was dessicated under reduced pressure(G8). After G8 was applied to Sephararose C1-4B by dissolving it in small amount of water, It was eluted with water and G9 was obtained from a tube No.25-30. G10 was obtained from the tube No.31-46.

Experiment 1

Anticancer test

Anactivity test on the sarcoma-180 of the fraction obtained in the above was conducted as follows :

The fraction was tansplanted at intervals of one week into the abdominal cavities of 20-25g ICR male mice and succesively transfered sarcoma-180 cells were used as experimental tumor cells.

The sarcoma-180 cells cultured for 7 days in the abdominal cavities of those mice were taken together with abdominal dropsy and it was centrifugally separated for 5 minutes by 4000 rpm after a sterile sale solution chilled with ice was added thereto, and a precipitate of cells was separated.

The separated cells were washed in a physiological saline solution three times and diluted so as to attain $1 \times 10^7$ cells/mg. 0.1ml of this cell suspension was transplanted hyperdomically into the left inguinal regions of a group of 10 mice.

Proteoglycans, which are purified fractions, were administered to those mice for 10 consecutive days 72 hours after tumor cells were transplanted. A physiological saline solution was administered to the control group and the proteoglycans dissolved in a physiological saline solution was administered to the test group 0.1ml by 0.1ml by concentrating it to 20mg/kg.

On the 30th day after transplantation of tumor cells, those mice were rendered lethal and induced solid cancer was extracted. Then, average tumor weight was found by measuring its weight and percent tumor inhibition ratio was calculated according to the formula given below.

$$I.R.\% = \frac{CW - TW}{CW} \times 100$$

I.R. = Percent inhibition ratio
CW = Average tumor weight of control group
TW = Average tumor weight of test group

As the result of an antitumor experiment conducted with sarcoma-180, G9 which was found most excellent in the control group as shown in Table 1 was picked up and designated as G009. Analysis thereof was made in the following experiments.

Table 1

| Anticancer effects produced by proteoglycan mice into which sarcoma-180 was transplanted | | |
|---|---|---|
| proteoglycan | average weight of tumors(g) (Mean±S.E) | inhibition ratio(%) |
| control | 4.66±0.17 | |
| G1 | 1.43±0.25 | 69.3 |
| G2 | 3.54±0.36 | 24.1 |
| G3 | 3.46±0.39 | 25.7 |
| G4 | 1.05±0.17 | 77.4 |
| G5 | 1.83±0.29 | 60.7 |
| G6 | 1.62±0.10 | 65.2 |
| G7 | 0.64±0.22 | 86.2 |
| G8 | 0.43±0.21 | 90.7 |
| G9 | 0.17±0.09 | 96.3 |
| G10 | 1.40±0.17 | 69.9 |

For chemical analysis of G009, the aggregate amount of saccharide was me asured by Anthrone's color developing method and protein content was measured by Lowry et al's method. Then, amino acid and saccharide were analyzed.

Results thereof are shown in Tables 2,3 and 4. A saccharide chromatogram is shown in Figure 1. An amino acid chromatogram is shown in Figure 2 and I.R a nalysis is shown in Figure 3.

G.L.C.(Shimadzu GC 9A, Japan) use requirements for analysis of saccharide :
Column 3% OV-17(80-100) (mesh shimalite)
3mmØ x 1 boronssilicate glass column
Temperature Column 150-180°C Gradient, Detector 190°C
Flow rate $N_2$ : 50ml/min
$H_2$ : 60ml/min(0.6kg/cm$^2$)
Air : 60ml/min(0.6kg/cm$^2$)
Attenuation $10^2 \times 2^1$ a.f.s(ampere full scale)

Table 2

| Total contents of polysaccharide and protein in G009 | | |
|---|---|---|
| Total content | polysaccharide (%) | protein (%) |
| G 009 | 93 | 5 |

Table 3

| Content of monosaccharide in the polysaccharide part of G 009. | |
|---|---|
| monosaccharide | Relative ratio(%) |
| $\alpha$-glucose | 45.84 |
| $\beta$-glucose | 41.70 |
| galactose | 8.03 |
| $\alpha$-mannose | 3.32 |
| fructose | 1.11 |

* Requirements for use of amino acid autoanalyzer(Beckman Sys. 6300. USA):

| | |
|---|---|
| Column | : 2.6x200mm |
| Ion exchange resin | : #338076(Beckman) |
| Flow rate | : Buffer solution 0.33ml/min |
| | Ninhydrin 0.17ml/min |
| Analysis cycle time | : 60 min |
| Column pressure | : 2100psi(147Kg/cm$^2$) |
| Ninhydrin pressure | : 100 psi(7Kg/cm$^2$) |
| Column temperature | : 50-70°C Gradient |
| N$_2$ gas pressure | : 40psi |
| Reaction bath temperature | : 130°C |
| Wave length | : 570 nm, 440 nm |

Table 4

| Total amino acid content in the protein part of G 009. | | | |
|---|---|---|---|
| Amino acids | $\mu$mol/ml | Amino acids | $\mu$mol/ml |
| Gly | 8.070 | Ile | 3.499 |
| Lys | 4.133 | Ser | 2.066 |
| Ala | 7.072 | Leu | 5.973 |
| His | 1.469 | Glu | 11.031 |
| Thr | 3.191 | Tyr | 1.638 |
| Arg | 0.796 | Pro | 6.446 |
| Val | 5.800 | Phe | 2.316 |
| Asp | 9.435 | Met | 0.938 |

Experiment 2

A variation of antitumor effects according to G009 administration routes

G 009 was transplanted into the abdominal cavities of 20-25g ICR male mice at intervals of one week and sarcoma-180 cells were used as experimental cells. The sarcoma-180 cells cultured for 7 days in the abdominal cavities of those mice were taken together with abdominal dropsy. It was centrifugally separated by 3000rpm for 5 minutes after adding a sterile saline solution chilled with ice thereto and a precipitate of cells was separated therefrom.

The separated cells were washed three times in a physiological saline solution and diluted so as to attain $1 \times 10^7$ cells/ml. 24 hours after the $1 \times 10^7$ cells/ml of cell suspension was transplanted into a group of 10 mice, 20mg/kg was injected into the muscle, subcutis, underthe skin and into the abdominal cavity 10 times every other day and 10mg/kg was injected into the vein 5 times every other day. A physiological saline solution was also administered 0.1ml by 0.1ml to the control group in the same manner. After the lapse of 30 days, weight of solid cancer was measured and compared.

As shown in Table 5, a cancerous growth was inhibited regardless of administration routes.

Table 5

| Comparison of antitumor effects according to the administration routes | | | |
|---|---|---|---|
| Administration route | Number of mice | Weight of tumor (test group/control) | Inhibition ratio(%) |
| Muscle | 10 | 0.22/4.46 | 95.1 |
| Subcutis | 10 | 0.28/4.52 | 93.8 |
| Abdomen | 10 | 0.18/4.72 | 96.1 |
| Vein | 10 | 0.21/4.45 | 95.2 |

Experiment 3

Life lengthening effects by G 009 administration

Ehrlich ascite tumor cells(EAT) were used to measure the antitumor activity of G 009 in the living body of mouse. $1 \times 10^6$ and $5 \times 10^7$ cells/ ml of EAT were injected into the abdominal cavity. After the lapse of 24 hours, G009 was dissolved in a phosphate buffer solution and 100mg/kg thereof was administered into the abdominal cavity and mouth for 12 consecutive days. Then, the average span of life for 20, 25, 30, 40 and 50 days was measured.

The same amount of physiological saline solution was administered to the control group at the same time.

As shown in Table 6, administration of G 009 into the abdominal cavity and vein produced a similar antitumor effect.

Table 6

| Life lengthening effects by G 009 administration | | | | | | | |
|---|---|---|---|---|---|---|---|
| | Dose (cells/ml) | Number of mice | Survival rate(%) during observing days after transplantation of cancer cells | | | | |
| | | | 20 | 25 | 30 | 40 | 50 |
| control | $1 \times 10^6$ | 10 | 100 | 40 | 10 | 0 | 0 |
| abdominal | $1 \times 10^6$ | 10 | 100 | 100 | 80 | 70 | 70 |
| intravenous | $1 \times 10^6$ | 10 | 100 | 90 | 70 | 60 | 60 |
| control | $5 \times 10^7$ | 10 | 100 | 30 | 0 | 0 | 0 |
| abdominal | $5 \times 10^7$ | 10 | 100 | 60 | 30 | 20 | 20 |

Experiment 4

Anticancer effects of G 009 in mice treated with trypan blue

G 009 was transplanted into the abdominal cavities of 20-25g ICR male mice at intervals of one week and sucessively transfered sarcoma-180 cells were used as experimental tumor cells. The sarcoma-180 cells cultured for 7 days in the abdominal cavity were taken together with abdominal dropsy. It was

6

centrifugally separated by 4000rpm for 5 minutes after adding thereto a sterile saline solution chilled with ice and a precipitate of cells was separated therefrom The separated cells were washed three times in a physiological saline solution and diluted so as to attain $1 \times 10^7$ cells/ml and hypodermically injected into the left inguinal region 0.1ml by 0.1ml.

After the lapse of one day, 4mg/kg(0.4ml) of trypan blue was administered into the abdominal cavity. Again, after the lapse of one day, 1mg/mouse(0.1ml) of trypan blue was injected hypodermically at a time at intervals of three days so as to amount to a total of 13mg/mouse.

Immediately after administratio of trypan blue, 10mg of G 009 was injected into the vein. Into the control group a physiological saline solution was injected instead of G009. Both control group and test group were comprised of 10 mice respectively. 30 days after transplantation of tumors, mice were rendered dead and weight of tumors was measured. Results of experiment conducted for antitumor effects of G009 in mice treated with trypan blue are shown in Table 7. These results reveal that G 009, a component extracted according to the present invention, activates macrophage associated with antitumor effects by the treatment of mice with trypan blue, a macrophage deactivating factor and that G 009 produces an antitumor effect by activating the function of macrophage together with tie antitumor activity of T-lymphocyte.

Table 7

| Anticancer effects of G 009 on mice treated with trypan blue. | |
|---|---|
| Group | weight of tumor(g) (Mean±S.E) |
| Control group<br>Group treated with G 009<br>Group treated with trypan blue<br>Group treated with G 009 and trypan blue | 4.54±0.26<br>0.14±0.05<br>4.81±0.19<br>2.69±0.17 |

Experiment 5

Effects produced when G 009 is administered into a newborn mouse and the mouse is treated with antithymic globulin after thymectomy

The thymectomy of a 2-day old ICR mouse was conducted according to Sjodin's method the cell suspension of sarcoma-180 was transplanted into the inguinal region of the mouse 0.1ml by 0.1ml concentrating it to $1 \times 10^7$ cells/ml after the lapse of 6 weeks from thymectomy.

After the lapse of 24 hours from transplantation, 20mg/kg of G 009 was injected into the muscle 10 times every other day. In order to prevent infection of the mouse which had thymectomy, water containing telracycline-HCl was fed to it. An antithymic serum was separated according to Tadakuma's method. Thymic cells were separated by cutting off the thymus from 1 2- or 3-week old mouse and made into cell suspension by diluting it in a phosphate buffer solution.

Then, $1 \times 10^6$ cells/ml of thymic cells were injected into the vein of a rabbit three times at intervals of three weeks for immunization. After the lapse of one week from the last injection, antithymic serum was taken and deactivated for 30 minutes at 56°C.

Afte the fraction of Ig G(immunoglobulin G) was separated from the deactivated antithymic serum by precipitating it with ammonium sulphate, 0.1ml thereof was injected for 10 days into the abdominal cavity of a mouse which passed one day after $1 \times 10^7$ cells/ml of sarcoma-180 was inoculated.

As to G009, 20mg/kg was injected into the muscle 5 times every other day after the lapse of one day from the transplantation of sarcoma-180.

As shown in Table 8 below, an antitumor effect produced by G 009 on the mouse the thymus of which was cut off in connection with the function of T-cell was remarkably lower than on the control group and an antitumor effect produced by G 009 on the group treated with antithymic globulin was reduced more than on the control group, a group pseudo-treated with normal rabbit globulin.

Thus, the results of these experiments are considered to reveal that G 009 produces an antitumor effect by activating the function of T-cells.

7

Table 8

| Comparison of antitumor effects produced by administration of G 009 after transplantation of sarcoma-180 into a newborn mouse who se thyms is resected(Experiment 1) and into a mouse treated with antithymic globulin (Experiment 2). | | | | | |
|---|---|---|---|---|---|
| | Treatment by kind | Number of mice | Dose (mg/Kg) | Wt. of tumor (Mean±S.E) | Inhibition ratio (%) |
| Experiment 1 | N.M<br>G 009 | 10<br>10 | -<br>20x10 | 4.75±0.37<br>0.18±0.06 | -<br>96 |
| | T.M<br>G 009 | 10<br>10 | -<br>20x10 | 3.55±0.55<br>2.70±0.07 | 25<br>43 |
| Experiment 2 | N.M<br>G 009 | 10<br>10 | -<br>20x5 | 5.40±0.31<br>0.25±0.04 | -<br>95 |
| | A.T.M<br>G 009 | 10<br>10 | -<br>20x5 | 3.78±0.59<br>3.16±0.59 | 30<br>41 |
| | N.R.G.M<br>G 009 | 10<br>10 | -<br>20x5 | 3.26±0.61<br>1.20±0.10 | 39<br>77 |
| * N.M : Normal mouse<br>T.M : Mouse the thymus of which was resected<br>A.T.M : Mouse treated with antithymic globulin<br>N.R.G.M : Mouse treated with normal rabbit globulin | | | | | |

Experiment 6

Effects produced by G 009 on the complement system

Gunia pig serums and fresh human serums were used as the complement. A sheep's red blood cell was used as a red blood cell. Antisheep-hemolysin was used as an antibody. Experiment on activating the complement was made as follows :

150ml of a gelatin veronal buffer solution(GVB$^{2+}$) and 50$\mu$l of a sample were put into a test tube, and 50$\mu$l of the complement (100 units/ml) was added thereto.

After it was made to react for 30 minutes at 37°C, the concentration of complement was adjusted to 1 unit/ml by adding GVB$^{2+}$ thereto.

After sheep's red blood cells sensitized in hemolysin(2MHU/ml) were added 2ml to these mixtures and 1.0, 1.2, 1.6 units were added respectively to the complement mixture adjusted with GVB$^{2+}$ solution, the total quantity was adjusted so as to amount to 5ml with GVB$^{2+}$.

The absorance of a supernatant solution obtained through centrifugal separation for 5 minutes by 2500rpm after reaction for 60 minutes at 37°C was measured at 541nm. The degree of activity was the amount(5) of the complement consumed by the control group and G 009.

As shown in Table 9, the amount of the complement consumed represented an increase in proportion to the concentration of G 009. Such an increase can be considered to reveal that G 009 increases immunity by activating the function of the complement which is one of the important protective functions of immune system.

Table 9

| Effects of G 009 on the activation of the compliment system | | |
|---|---|---|
| | conc ($\mu$g/ml) | Amount of the comsumed complement(%)(Mean±S.D) |
| G 009 | 10 | 15.2±0.59 |
| | 50 | 27.9±0.43 |
| | 100 | 36.4±0.1 |
| | 200 | 45.5±0.3 |
| | 600 | 52.2±0.7 |

Experiment 7

Effects produced by G 009 on the weight of organs associated with immunity

After the lapse of 5 days from intravenous injection of G 009 into the veins of mice in a dosage amounting to 10mg/kg, the lungs, the livers and the spleens were taken out and their weight was measured. Lysozyme was made to be reactive with streptococcus lysodeicus dissolved in a phosphate buffer solution-(pH 6.2) as a substrate and its activity was measured by absorbance at 660nm.

As shown in Table 10, the reason that the weight of the spleen and the degree of activity of lysozyme registered an increase in the group treated with G 009 was that G 009 activated macrophage and the activity value of lysozyme was thereby made to register an increase.

Table 10

| Effects of G 009 administration on the activity value of serum enzyme amd on the weight of organs | | | | | | |
|---|---|---|---|---|---|---|
| | Number of mice | Weight (g) | Liver (%) | Spleen (%) | Lungs (%) | Lysozyme ($\mu$g/ml) |
| None | 10 | 24.8±1.0 | 6.54±0.12 | 0.37±0.01 | 0.56±0.02 | 8.75±0.23 |
| G 009 | 10 | 24.5±1.2 | 6.60±0.21 | 0.42±0.04 | 0.54±0.07 | 10.01±0.48 |
| * Weight of each organ is a relative value to the weight of a mouse. | | | | | | |

Experiment 8

Effects of G 009 on the number of hemolytic plaque forming cells

1). A group of 5 ICR male mice weighing 20-25g was used as laboratory animals and a sample concentrated to 20mg/kg was injected into their abdominal cavities for 5 consecutive days. After the lapse of 7 days from the last administration, they were immunized by injecting sheep's red blood cells into their abdominal cavities in concentration amounting to $1 \times 10^6$ cells/ml. Then, the spleen was taken out 4 days after sheep's red blood cells were administered thereinto.

The spleen cells were extricated by triturating the spleen with a blender together with an equilibrium salt solution chilled with ice. The extricated spleen cells were suspended in a 0.83% ammonium chloride solution and left as they were for 3 minutes at 37°C to remove a supernatent solution through centrifugal separation for 5 minutes by 2000rpm. Those cells were again suspended in the equilibrium salt solution chilled with ice through centrifugal separation and the number of the spleen cells was measured with a hemometer.

2). The sheep's red blood cells suspended in Alser's solution(20.5g of glucose, 4.2g of sodium chloride and 8.0g od sodium citrate were dissolved in 1 liter of distilled water and used by filtering it with a millipore fiter(0.45$\mu$m)) were washed four times in the equilibrium salt solution for 5 minutes by 2000rpm and suspended in the same solution so as to amount to 10% in final concentration.

3). A basement plate was made by pouring 10ml of 1.5% agar(Noble agar, Difco) into a petri dish. Again, 100$\mu$l of 1) and 100$\mu$l of sheep's red blood cells of 2) were mixed into 2ml of 0.7% agar and poured into the petri dish to attain uniform spread. After it was sensitized for 60 minutes at 37°C, moisture-absorbed guinea pig serum were diluted ten fold with an equilbrium salt solution as the complement and added to it 2.5ml by 2.5ml and cultured for 30 minutes at 37°C. Then, the number of formed hemolytic plaque forming cells and the number of hemolytic plaque forming cells among all spleem cells(PFC/spleen) were calculated.

As shown in Table 11 below, G 009 cause the number of spleen cells and the number of hemolytic plaque forming cells to increase remarkably. Thus, G 009 is considered to be associated with an immunostimulating action.

Table 11

| Effects of G 009 on hemolytic plaque forming cells in the spleens of mice immunized with sheep's red blood cells | | | |
|---|---|---|---|
| | Noumber of mice | Number of spleen cells($1 \times 10^7$) | PFC/the spleen cells ($1 \times 10^6$) |
| Control | 5 | 38.6±2.1 | 24.4±11.2 |
| G 009 | 5 | 42.6±0.11 | 51.0±14.5 |

Experiment 9

Immunostimulating action

Each group comprises 10 mice. 6-week old, 20-25g, male, ICR mice were used. G 009 was dissolved in a physiological saline solution and 0.2ml of solution by concentration was administered into their abdominal cavities, 24 hours later, 0.2ml of carbon suspension manufactured by mixing 1ml of perikan drawing ink 17 black and 2ml of physiological saline solution was injected into the veins of their tails. Then, at 1, 5, 10 and 15 minutes, 0.02ml of blood was taken from the orbit of an eye with a hematocrit capillary tube coated with heparin and immediately diluted and hemolyzed with 1.6ml of an aqueous solution of sodium xarbonate. This solution was colorimetered at 675nm and a phagocytosis index(K value) was measured according to Halpern et al's aquation. In the control group, 0.2ml of physiological saline solution was administered to the mouse.

$$K = \frac{\log C_0 - \log C}{t - t_0}$$

In the above equation,
    $C_0$ is the content of carbon powder in blood at time $t_0$.
    $C$ is the content of carbon powder in blood at time $t$.

Results of these experiments are shown in Table 12 below. An index(K) of immunophagocytosis was registered high in proportion to the concentration of G 009. According to those results, G 009 appears most likely to produce an immunostimulating effect by playing a part in immunoresponse.

Table 12

| Effects of G 009 on immunostimulating action. | | | |
|---|---|---|---|
| | Dose (mg/Kg) | Number of mice | Index of phagocytosis (K) (Mean±S.E) |
| control | saline solution | 10 | 0.0391±0.003 |
| G 009 | 5 | 10 | 0.1109±0.025 |
| | 20 | 10 | 0.1453±0.022 |
| | 50 | 10 | 0.1643±0.008 |
| | 100 | 10 | 0.1845±0.011 |

Experiment 10

Toxicity test of G 009

Acute toxicity test of G 009 was conducted on mice, rats and rabbits and results thereof were measured after the lapse of 14 days from administration of G 009.

As shown in Table 13 below, there was no mortality found through routes and species. Therefore, G 009 is a safe material remarkably low in toxicity.

Table 13

| Results of toxicity test of G 009 | | | | | |
|---|---|---|---|---|---|
| Tested animal | Number of mice | Dose (mg/Kg) | Route | Mortality | Maximum allowance(mg/kg) |
| Mouse | 10 | 100 - 300 | i.v | 0 | > 300 |
| | 10 | 50 - 2000 | i.p | 0 | >2000 |
| | 10 | 50 - 100 | i.m | 0 | > 100 |
| | 10 | 1000 - 2000 | s.c | 0 | >2000 |
| Rat | 10 | 100 - 300 | i.v | 0 | > 300 |
| | 10 | 500 -1000 | i.p | 0 | >1000 |
| | 10 | 50 - 100 | i.m | 0 | > 100 |
| | 10 | 500 -1000 | s.c | 0 | >1000 |
| Rabbit | 10 | 100 - 200 | i.v | 0 | > 200 |
| | 10 | 100 - 200 | i.p | 0 | > 200 |
| | 10 | 25 - 50 | i.m | 0 | > 50 |
| | 10 | 50 - 100 | s.c | 0 | > 100 |

Experiment 11

Comparison of characteristics between the species.

In order to ascertain the phylogenetic classification and physiological genetic differences of four kinds of type strains(retained by Bacteria 2 section, Agricultural Technology Institute, Office of Rural Development) found excellent as the result of a search made for an anticancer effect produced by those ganoderma lucodum strains collected in various areas of the country, the following experiments were conducted.

Experimental materials and methods:

Used strains, culture medium and culture

The strains used for the present experiment were four strains found excellent in anticancer effect among those ganoderma lucidums which grew naturally in the country and the places of collection are shown in Table 14.

Extraction of a sample

The mycelia obtained by centrifugal separation of those cultured in liquid were washed three times in a phosphate buffer saline solution(pH 7.5)n and treated with supersonic waves for 30 seconds under ice-cooling.

After centrifugal separation at 12000xg, its supernatent solution was used as a sample for electrophoresis.

Quantification of proteins

Proteins in sample were quantified with bovine serum albumin(BSA) used as a type material and by using a protein assay reagent(Pierce Co.).

Electrophoresis

A discontinuous buffer system was used for electrophoresis. Separating gel was prepared by concentrating it to 10% C, 10% T in 240nM Tris-Cl buffer solution(pH 8.48) and stacking gel was prepared by concentrating it to 3.125% T and 20% C in a Tris buffer solution(39.5mM Tris, 0.064N $H_3PO_4$, pH 6.9).

In order to solidify these gels, TEMED and ammonium persulfate were used. As running buffers, 40mM Tris-glycin buffer solution(pH 8.8) was used at anode and 60mM Tris-Cl buffer solution(pH 7.47) was used at cathode. The sample was developed for 2 hours with 100V at 4°C by loading it on the gels 70$\mu$g by 70$\mu$g.

Gel staining

(1). Esterase(E.C. 3,1,1,1)

The developed gels were digested in 0.2M phosphate buffer solution(pH 6.5) for 30 minutes. While those gels were being digested, said solution was replaced for a new solution three times. After the activity of digestion-finished gels was adjusted, a color developing solution($\alpha$-naphthylacetate 20mg, ethylene glycolmonoethyl ether 2ml, fast blue RR salt 20mg, 0.2M phosphate buffer 120ml) was added thereto and shaked at 35°C in the dark place for color development.

(2). Acid phosphatase(E.C. 3,1,3,2)

After activity in gel was adjusted by digesting it in 0.1M acetate buffer solution(pH 5.2), a color developing solution(10% $MgCl_2$ solution 6ml, fast garnet GBC salt 70mg, $\alpha$-naphthylphosphate 80mg, $\beta$-naphthylphosphate 40mg, 0.1M acetate buffer 100ml) was added thereto for color development for 30 minutes at 37°C.

(3). Leucine amino peptidase(E.C 3,4,11,1)

A color developing solution(L-leucyl-$\beta$-naphthylamide HCl 20mg, fast blue K salt 20mg, distilling water 50ml, 0.2M Tris-malate buffer(pH 5.4) 120ml) was added to the gels for color development for 30 minutes in the dark place.

(4). Peroxidase(E.C. 1,11,1,7)

After the gels were washed in water, a color developing solution(Benzidine 1%, acetic acid 9ml, mixed solution of 1 part of benzidine solution mixed with 40ml of distilled water, 1 part of 0.03% $H_2O$ and 4 part of distilled water) was added therto for color development in the dark place.

Results of these experiments were as follows:

- Iseozyme pattern of esterase -

As to the esterase bands of ganoderma lucidum, 17 bands came forth in all as shown in Fig 1. The 5th and 15th bands were common to all strains. The esterase pattern of type strains Fr 07004, Fr 07008 and IY 005 were very similar to one another but the esterase pattern of IY 009 and IY 010 were greatly different from others.

- Isoenzyme pattern of acid phosphatase -

As shown in Figure 5, the acid phosphatase pattern of type strains Fr 07004, Fr 07008 and IY 005 were very similar to one another but IY 009 and IY 010 showed a different band pattern from others.

- Isoenzyme pattern of leucine amino peptidase -

From ganoderma lucidum, 3 leucine amino peptidase bands came forth in all. Although those type strains Fr 07004, Fr 07008 and IY 005 showed the same pattern for tile most part, IY 009 showed a pattern entirely different from them(Figure 5).

- Isoenzyme pattern of peroxidase -

2 peroxidase bands came forth from the strain of ganoderma lucidum and there was a great affinity among type strains Fr 07004, Fr 07008 and IY 005(Figure 5).

- Affinity between the species -

As shown in Table 15, an affinity between type strains Fr 07008 and IY 005 represented 93.8% and an affinity between type strain Fr 07004 and IY 005 showed 82.4%, but an affinity between Fr 07004 and IY 010 and between Fr 07004 and IY 009 registered 30.4% and 31.8%.

According to the above results, a difference of isoenzyme pattern in the same species was made due to a biochemical variation caused by a genetic mutation resultant from a difference of geographical environment and such a difference seemed most likely to be associated with the fact that every strain creates a proteoglycan different in anticancer activity.

Table 14

| Areas wherein those strains used for the present experiment were collected | | | |
|---|---|---|---|
| Species | A number of strain | Areas wherein strains were collected | Note |
| Ganoderma lucidum | Fr 07004<br>Fr 07008 | Office of Rural Development<br>Office of Rural Development | Type strain<br>Type strain |
| | IY 005 | Kwangnung,<br>Kyung-gi Province | |
| | IY 008 | Dooryoon Mountains,<br>South Cholla Province | |
| | IY 009 | Dooryoon Mountains,<br>South Cholla Province | |
| | IY 010 | Chiak Mountains, Kangwon Province | |

Table 15

| Comparison of an affinity between the strains of Ganoderma lucidum according to the pattern esterase, leucine amino peptidase and peroxidase | | | | | | | |
|---|---|---|---|---|---|---|---|
| Species | A number of strain | Affinity (%) | | | | | |
| | | FR 07004 | Fr 07008 | IY 005 | IY 008 | IY 010 | IY 009 |
| Ganoderma lucidum | Fr 07004 | * | 77.8 | 82.4 | 62.5 | 30.4 | 31.8 |
| | Fr 07008 | | * | 93.8 | 45.0 | 32.8 | 25.0 |
| | IY 005 | | | * | 45.0 | 31.8 | 25.0 |
| | IY 008 | | | | * | 27.3 | 40.0 |
| | IY 010 | | | | | * | 25.0 |
| | IY 009 | | | | | | * |

## Claims

1. Ganoderma lucidum IY 009 which produces a proteoglycan G 009 possessed of antitumoral and immunostimulating effects, and separated from the cultured Ganoderma lucidum, containing:
   $\beta$-glucose, $\alpha$-glucose, galactose, $\alpha$-mannose and fructose as saccharide components, and glycine, alanine, histidine, arginine, valine, aspartic acid, threonine, isoleucine, serine, leucine, glutamic acid, tyrocine, proline, phenylalanine and methionine as protein components.

14

# INTERNATIONAL SEARCH REPORT

International Application No  PCT/KR91/00032

| I. CLASSIFICATION OF SUBJECT MATTER (If several classification symbols apply, indicate all) [6] |
|---|
| According to International Patent Classification (IPC) or to both National Classification and IPC |
| Int. Cl[5]   C12N1/14//C12P21/00, (C12N1/14, C12R1:645),<br>(C12P21/00, C12R1:645) |

| II. FIELDS SEARCHED |
|---|

| Minimum Documentation Searched [7] |
|---|

| Classification System | Classification Symbols |
|---|---|
| IPC | C12N1/14, C12P21/00 |

| Documentation Searched other than Minimum Documentation<br>to the Extent that such Documents are Included in the Fields Searched [8] |
|---|
| Biosis Database, Chemical Abstract Database<br>(CA, REG) |

## III. DOCUMENTS CONSIDERED TO BE RELEVANT [9]

| Category [*] | Citation of Document, [11] with indication, where appropriate, of the relevant passages [12] | Relevant to Claim No. [13] |
|---|---|---|
| Y | J, Chin. Agric. Chem. Soc., Vol. 26, No. 4, 1988, Hseu R-S. et al. "Identification of Ganoderma-Iucidum and Ganoderma-tsugae by cultural studies and the incompatibility test"  p. 588-596 | 1 |
| Y | J. Pharmaco bio-Dyn., Vol. 12, No. 2, 1989, Maruyama H. et al. "Antitumor activity of Sarcodon-aspratus berk. S. ito and Ganoder-malucidum Fr. Karst"  p. 118-124 | 1 |
| Y | Nippon Nogeikagaku kaishi, Vol. 59, No. 11, 1985, Mizuno T. et al. "Fractionation chemical modification and antitumor activity of mater-insoluble polysaccharides of the fruiting-body of Ganoderma-Incidum"  p. 1143-1152 | 1 |
| Y | Nippon Nougeikagaku Kaishi, Vol. 58, No. 9, 1984, Mizuno T. et al. "Fractionation Structural features and antitumor activity of water-soluble polysaccharide from reishi the fruitbody of Ganoderma-lucidum" | 1 |

* Special categories of cited documents: [10]

"A"  document defining the general state of the art which is not considered to be of particular relevance

"E"  earlier document but published on or after the international filing date

"L"  document which may throw doubts on priority claim(s) or which is cited to establish the publication date of another citation or other special reason (as specified)

"O"  document referring to an oral disclosure, use, exhibition or other means

"P"  document published prior to the international filing date but later than the priority date claimed

"T"  later document published after the international filing date or priority date and not in conflict with the application but cited to understand the principle or theory underlying the invention

"X"  document of particular relevance; the claimed invention cannot be considered novel or cannot be considered to involve an inventive step

"Y"  document of particular relevance; the claimed invention cannot be considered to involve an inventive step when the document is combined with one or more other such documents, such combination being obvious to a person skilled in the art

"&"  document member of the same patent family

| IV. CERTIFICATION |
|---|

| Date of the Actual Completion of the International Search | Date of Mailing of this International Search Report |
|---|---|
| March 12, 1992 (12. 03. 92) | March 31, 1992 (31. 03. 92) |

| International Searching Authority | Signature of Authorized Officer |
|---|---|
| Japanese Patent Office | |

Form PCT/ISA/210 (second sheet) (January 1985)

**FURTHER INFORMATION CONTINUED FROM THE SECOND SHEET**

|   |   |   |
|---|---|---|
| | p. 871-880 | |
| Y | Chem. Pharm. Bull., Vol. 29, No. 12, 1981, Miyazaki T. et al. "Studies on fungal poly saccharides 27. structural examination of a water soluble anti tumor poly saccharido of Ganoderma-lucidum"  p. 3611-3616 | 1 |

V.☐ **OBSERVATIONS WHERE CERTAIN CLAIMS WERE FOUND UNSEARCHABLE** [1]

This international search report has not been established in respect of certain claims under Article 17(2) (a) for the following reasons:

1.☐ Claim numbers        . because they relate to subject matter not required to be searched by this Authority, namely:

2.☐ Claim numbers        , because they relate to parts of the international application that do not comply with the prescribed requirements to such an extent that no meaningful international search can be carried out, specifically:

3.☐ Claim numbers        , because they are dependent claims and are not drafted in accordance with the second and third sentences of PCT Rule 6.4(a).

VI.☐ **OBSERVATIONS WHERE UNITY OF INVENTION IS LACKING** [2]

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

Form PCT/ISA/210 (supplemental sheet (2)) (January 1985)